# EUROPÄISCHE PATENTSCHRIFT

(11) **EP 2 482 926 B1**
(45) Veröffentlichungstag und Bekanntmachung des Hinweises auf die Patenterteilung: **28.05.2014**
(21) Anmeldenummer: 10748049.3
(22) Anmeldetag: 12.08.2010
(51) Int. Cl.: A61N 5/10

(54) **VERFAHREN UND VORRICHTUNG ZUR ÜBERPRÜFUNG EINER BESTRAHLUNGSPLANUNG SOWIE BESTRAHLUNGSANLAGE**
METHOD AND DEVICE FOR CHECKING AN IRRADIATION PLANNING SYSTEM, AND IRRADIATION SYSTEM
PROCÉDÉ ET DISPOSITIF POUR VÉRIFIER UN PROGRAMME DE TRAITEMENT PAR RAYONNEMENT ET INSTALLATION DE TRAITEMENT PAR RAYONNEMENT

(30) Priorität: 29.09.2009 DE 102009043283
(43) Veröffentlichungstag der Anmeldung: 08.08.2012
(73) Patentinhaber: GSI Helmholtzzentrum für Schwerionenforschung GmbH, 64291 Darmstadt (DE); Siemens Aktiengesellschaft, 80333 München (DE)
(72) Erfinder: BERT, Christoph, 63741 Aschaffenburg (DE); RIETZEL, Eike, 64331 Weiterstadt (DE); SAITO, Nami, 64291 Darmstadt (DE)
(74) Vertreter: Mergel, Volker
(86) Internationale Anmeldenummer: PCT/EP2010/004956
(87) Internationale Veröffentlichungsnummer: WO 2011/038802

(56) Entgegenhaltungen:
- WO-A2-2004/103145
- US-A1- 2008 298 540
- VINOGRADSKIY YEVGENIY Y ET AL: "Verification of four-dimensional photon dose calculations." MEDICAL PHYSICS, Bd. 36, Nr. 8, August 2009 (2009-08), Seiten 3438-3447, XP002605342 ISSN: 0094-2405
- MINOHARA S ET AL: "RESPIRATORY GATED IRRADIATION SYSTEM FOR HEAVY-ION RADIOTHERAPY", INTERNATIONAL JOURNAL OF RADIATION: ONCOLOGY BIOLOGY PHYSICS, PERGAMON PRESS, USA, vol. 47, no. 4, 1 January 2000 (2000-01-01), pages 1097-1103, XP001051093, ISSN: 0360-3016, DOI: 10.1016/S0360-3016(00)00524-1

## Beschreibung

Die Erfindung betrifft ein Verfahren und eine Vorrichtung zur Überprüfung einer Bestrahlungsanlage sowie eine Bestrahlungsanlage mit einer derartigen Vorrichtung. Derartige Verfahren und Vorrichtungen werden insbesondere zur Qualitätsüberprüfung einer Bestrahlungsanlage eingesetzt, und können z.B. im Vorfeld einer geplanten Bestrahlung eingesetzt werden, um das korrekte Funktionieren der Anlage zu überprüfen.

Die Partikeltherapie ist ein etabliertes Verfahren zur Behandlung von Gewebe, insbesondere von Tumorerkrankungen. Bestrahlungsverfahren, wie sie in der Partikeltherapie eingesetzt werden, finden jedoch auch in nichttherapeutischen Gebieten Anwendung. Hierzu gehören beispielsweise Forschungsarbeiten, etwa zur Produktentwicklung, im Rahmen der Partikeltherapie, die an nicht-lebenden Phantomen oder Körpern durchgeführt werden, Bestrahlungen von Materialien, etc.

Hierbei werden geladene Partikel wie z.B. Protonen oder Kohlenstoffionen oder andere Ionen auf hohe Energien beschleunigt, zu einem Partikelstrahl geformt und über ein Hochenergiestrahltransportsystem zu einem oder mehreren Bestrahlungsräumen geführt. In dem Bestrahlungsraum wird das zu bestrahlende Zielvolumen mit dem Partikelstrahl bestrahlt.

Es kann dabei vorkommen, dass sich das zu bestrahlende Zielvolumen bewegt. Beispielsweise kann bei der Bestrahlung eines Patienten eine Bewegung des zu bestrahlenden Tumors durch eine Atembewegung verursacht werden.

Eine bekannte Möglichkeit, die Bewegung des Zielvolumens zu kompensieren, sind Bestrahlungsverfahren, die unter den Begriffen "Rescanning", "Gating" und "Tracking" bekannt sind. Unter "Rescanning" wird verstanden, dass der Strahl mehrfach wie geplant appliziert wird, so dass sich Fehldosierungen der einzelnen Durchläufe ausmitteln. Unter "Gating" wird verstanden, dass der Strahl nur zu festgelegten Zeitfenstern der Bewegung appliziert wird, so dass der Bewegungseinfluss gemindert oder bestenfalls sogar ausgeschlossen wird. Unter "Tracking" wird verstanden, dass der Strahl, mit dem die Bestrahlung des Zielvolumens erfolgt, der Bewegung des Zielvolumens nachgeführt wird. Falls der Strahl ein Partikelstrahl ist, kann dies beispielsweise erreicht werden, indem der Strahl durch Magnetsysteme derart abgelenkt wird, dass sein Verlauf der Bewegung des Zielvolumens nachgeführt wird. Gegebenenfalls kann der Strahl auch in seiner Energie variiert werden, um die Eindringtiefe des Strahls der Bewegung des Zielvolumens anzupassen. Auch bei einer Bestrahlung mit Photonen kann ein Tracking durchgeführt werden. Dies kann z.B. durch eine Veränderung des den Strahl begrenzenden Kollimators erfolgen, wobei die Kollimatoröffnung an die Bewegung des Zielvolumens angepasst wird.

Aus der US 6,891,177 B1, der US 6,710,362 B2 und der US 2006/0033042 A1 sind verfahren und Vorrichtungen bekannt, mit denen eine Bewegungsnachführung des Strahls durchgeführt werden kann.

All diese Kompensationsverfahren können im Rahmen der Partikeltherapie bei einem sogenannten Scan-Verfahren angewendet werden, bei dem eine Vielzahl von räumlich eng umschriebenen Bestrahlungsdosen sukzessive an unterschiedlichen Orten im Zielvolumen deponiert werden, bei dem also der Partikelstrahl über das Zielvolumen gescannt wird.

Es ist die Aufgabe der Erfindung, ein Verfahren zur Überprüfung einer Bestrahlungsanlage, insbesondere einer Partikeltherapieanlage, und/oder einer insbesondere patientenspezifischen Bestrahlungsplanung bereitzustellen, welches ein sicheres Betreiben der Bestrahlungsanlage selbst bei der Bestrahlung von bewegten Zielvolumina erlaubt. Weiterhin ist es die Aufgabe der Erfindung, eine Vorrichtung zur Überprüfung einer derartigen Anlage bereitzustellen sowie eine Bestrahlungsanlage zur Durchführung eines derartigen Verfahrens.

Die Aufgabe wird durch die Merkmale der unabhängigen Ansprüche gelöst. Bevorzugte Ausgestaltungen der Erfindung sind in den abhängigen Ansprüchen angegeben und werden im Folgenden näher erläutert.

Die vorangehende und die folgende Beschreibung der einzelnen Merkmale bezieht sich sowohl auf die Vorrichtungskategorie als auch auf die Verfahrenskategorie, ohne dass dies im Einzelnen in jedem Fall explizit erwähnt ist; die dabei offenbarten Einzelmerkmale können auch in anderen als den gezeigten Kombinationen erfindungswesentlich sein.

Das erfindungsgemäße Verfahren zur Überprüfung einer Bestrahlungsanlage, insbesondere einer Partikeltherapieanlage, in der mit einem Behandlungsstrahl eine Dosisverteilung in einem Zielobjekt deponierbar ist, umfasst folgende Schritte:
- Bereitstellen eines Bestrahlungsplanungsdatensatzes, der für die Bestrahlung eines sich bewegenden Zielvolumens ausgelegt bzw. optimiert ist,
- Bereitstellen eines Bewegungssignals, das eine Bewegung des Zielvolumens nachbildet,
- Bestrahlen eines Phantoms, das zur Detektion einer in dem Phantom deponierten Dosisverteilung während oder nach der Bestrahlung ausgebildet ist, wobei das Phantom unter Verwendung der im Bestrahlungsplanungsdatensatz hinterlegten Steuerparameter und des Bewegungssignals bestrahlt wird,
- Ermitteln einer im Phantom deponierten Dosisverteilung,
- Berechnen einer zu erwartenden Dosisverteilung aufgrund von Parametern, die im Zusammenhang mit der Steuerung der Bestrahlungsanlage während der Bestrahlung stehen,
- Vergleichen der im Phantom deponierten ermittelten Dosisverteilung mit der berechneten zu erwartenden Dosisverteilung.

Dem erfindungsgemäßen Verfahren liegt die Idee zu Grunde, dass die Qualitätssicherung für die Bestrahlung eines sich bewegenden Zielvolumens wesentlich schwieriger durchzuführen ist als bei der Bestrahlung eines statischen Zielvolumens, da mit der Bewegung ein weiterer Parameter hinzukommt, der prinzipiell zu Fehlern während der Bestrahlung führen kann. Insbesondere ist die Bewegung des Zielvolumens ein Faktor, der erst während der Bestrahlung bestimmt werden kann und nicht genau vorhergesagt werden kann.

Es wird vorgeschlagen, dass einerseits ein Phantom bei der Qualitätsüberprüfung bestrahlt wird, so dass in dem Phantom eine Dosisverteilung gemäß einem Bestrahlungsplanungsdatensatz und einem bereitgestellten Bewegungssignal deponiert wird.

Andererseits wird berechnet, welche Dosisverteilung in dem Phantom zu erwarten ist. Für diese Berechnung können Daten verwendet werden, die zur Steuerung der Bestrahlungsanlage während der Bestrahlung verwendet werden, also Parameter, die beispielsweise im Bestrahlungsplanungsdatensatz hinterlegt sind und die direkt oder indirekt zur Steuerung der Bestrahlungsanlage verwendet werden, sowie Daten, die während der aktuellen Bestrahlung protokolliert werden und die den Verlauf der Bestrahlung und insbesondere die aktuellen Eigenschaften des Strahls während der Bestrahlung kennzeichnen. Diese Daten umfassen in der Regel auch die zeitlich zur Bestrahlung korreliert aufgenommene Bewegungstrajektorie.

Die berechnete, zu erwartende Dosisverteilung wird der Dosisverteilung, die anhand des Phantoms gemessen bzw. ermittelt wird, gegenübergestellt und somit mit dieser verglichen. Durch den Vergleich der zu erwartenden Dosisverteilung mit der tatsächlich im Phantom applizierten und anhand des Phantoms ermittelten Dosisverteilung kann festgestellt werden, ob die Bestrahlung auch dann korrekt durchgeführt wird, wenn das Bestrahlungsverfahren die Bewegung eines Zielvolumens kompensiert. Die Berechnung der Dosisverteilung bei einer Partikeltherapieanlage umfasst insbesondere die Berechnung der Eindringtiefe des Partikelstrahls in das Zielobjekt, insbesondere in das Zielvolumen und gegebenenfalls auch um das Zielvolumen angeordnete Bereiche. Insbesondere wenn die berechnete erwartete Dosisverteilung der ermittelten Dosisverteilung entspricht, also mit dieser in etwa übereinstimmt, kann eine gute Funktion der Bestrahlungsvorrichtung bestätigt werden und dadurch der Bestrahlungsplan validiert werden.

Die Bestrahlung des Phantoms findet dabei derart statt, dass eine Bewegungskompensation basierend den im Bestrahlungsplanungsdatensatz hinterlegten Steuerparametern, meist in Kombination mit dem Bewegungssignal, durchgeführt wird. Das Phantom wird also so bestrahlt, als ob es sich gemäß dem Bewegungssignal bewegen würde. Das Bewegungskompensationssystem der Anlage steuert die Bestrahlung der Anlage derart, als ob sich ein Zielvolumen nach dem Bewegungssignal bewegen würde. Das Bewegungskompensationssystem der Anlage kann dabei unterschiedlich ausgebildet sein und beispielsweise ein Tracking-Verfahren und/oder ein Gating-Verfahren und/oder ein Rescanning-Verfahren implementieren. Das Bewegungskompensationssystem minimiert folglich den dosimetrischen Einfluss der Bewegung eines Zielvolumens.

Das Phantom ist dabei derart ausgebildet, dass während oder nach der Bestrahlung des Phantoms die in dem Phantom deponierte Dosis, genauer die in dem Phantom deponierte räumliche Verteilung der Dosis, ermittelt bzw. gemessen werden kann. In einem einfachen Fall kann das Phantom ein zweidimensionaler Film sein, der bei der Bestrahlung mit dem Behandlungsstrahl geschwärzt wird. Durch Auswertung des Films nach der Bestrahlung kann die Dosisverteilung zweidimensional ausgewertet und dadurch ermittelt werden. Die so ermittelte Schwärzung kann mit der zu erwartenden Dosisverteilung bzw. Schwärzung des Films verglichen werden. Vorzugsweise ist das Phantom jedoch dreidimensional aufgebaut um die Dosisverteilung dreidimensional ermitteln zu können. Zum Beispiel kann in einem dreidimensionalen Körper eine Vielzahl von Dosismesskammern integriert sein. Durch Auswertung der in den Dosismesskammern generierten Signale wird die in dem Phantom deponierte Dosisverteilung gemessen. Es kann auch ein Phantom eingesetzt werden, das biologisches Zellmaterial umfasst, sodass die Dosisverteilung bzw. der Einfluss der Bestrahlung durch Auswertung des bestrahlten Zellmaterials erfolgen kann.

Das Phantom kann dabei homogen aufgebaut sein - genauer ist das Material, in welchem die Dosismesskammern integriert sind, homogen. Das Material kann jedoch auch inhomogen sein mit verschiedenen Bereichen, die eine unterschiedliche Eindringtiefe für einen Partikelstrahl aufweisen. Damit kann beispielsweise der Aufbau eines menschlichen Körpers mit unterschiedlichen Organen und Geweben simuliert werden (antrophomorphes Phantom).

Der Bestrahlungsplanungsdatensatz kann ein dreidimensionaler und insbesondere ein vierdimensionaler Bestrahlungsplanungsdatensatz sein. Letzterer bedeutet, dass der Bestrahlungsplanungsdatensatz die zeitliche Dimension und die Bewegung des Zielvolumens berücksichtigt. Ein derartiger Bestrahlungsplanungsdatensatz basiert üblicherweise auf einem vierdimensionalen Abbildungsdatensatz, der die Bewegung des Zielvolumens abbildet. Anhand des vierdimensionalen Abbildungsdatensatzes kann die Dosisverteilung im Zielvolumen und deren notwendige Anpassung geplant werden, um die gewünschte Bestrahlung trotz Bewegung des Zielvolumens sicherstellen zu können. Ein Beispiel für einen derartigen vierdimensionalen Bestrahlungsplanungsdatensatz ist in der US 2009/0095921 A1 offenbart, welche einen vierdimensionalen Bestrahlungsplanungsdatensatz für ein Scanverfahren einer Partikeltherapieanlage beschreibt, bei dem der Partikelstrahl der Bewegung des Zielvolumens nachgeführt wird. Der Bestrahlungsplanungsdatensatz umfasst die Dosisverteilung und die dazu notwendigen Strahlparameter für eine Referenzphase sowie die Kompensationsparameter für andere Bewegungsphasen, mit denen die Strahlparameter der Referenzphase verändert werden, um die korrekte Dosisdeposition in der anderen Bewegungsphase zu gewährleisten. Ein vierdimensionaler Bestrahlungsplanungsdatensatz kann zusammen mit dem Bewegungssignal während einer Bestrahlung die Bestrahlung derart steuern, dass die gewünschte Dosisverteilung im Zielvolumen trotz Bewegung des Zielvolumens erreicht wird.

Der vierdimensionale Bestrahlungsplanungsdatensatz kann auf einer Bestrahlungsplanungsvorrichtung erstellt werden und in eine Steuerungsvorrichtung der Bestrahlungsanlage geladen werden. Anhand der Parameter, die im Bestrahlungsplanungsdatensatz hinterlegt sind, kann die Steuerungsvorrichtung die Strahlapplikation und insbesondere die Bewegungskompensation bei der Strahlapplikation steuern. Als Bewegungskompensation kann insbesondere eine Bewegungsnachführung des Strahls durchgeführt werden.

Die Bewegungsnachführung während der Bestrahlung wird aufgrund eines Bewegungssignals durchgeführt. Da nicht das Zielvolumen bestrahlt wird, für das die Bestrahlungsplanung durchgeführt wurde, sondern ein Phantom, wird ein Bewegungssignal bereitgestellt, das die Bewegung des Zielvolumens nachbildet. Dies bedeutet, dass das Bewegungssignal lediglich ein Signal wiedergibt, dass potentiell während einer Bestrahlung des Zielvolumens auftreten kann. Die Bewegung des Zielvolumens wird also durch das Bewegungssignal simuliert.

Das Bewegungssignal kann beispielsweise erzeugt werden, indem eine Bewegungsdetektionsvorrichtung wie z.B. ein externer Sensor, der während einer Bestrahlung des Zielvolumens die Bewegung überwacht, durch einen sich bewegenden physikalischen Gegenstand stimuliert wird. Ist der externer Sensor beispielsweise ein optischer Sensor, mit dem die Bewegung eines auf einen Patienten angebrachten Marker verfolgt werden kann, kann durch eine Bewegungsvorrichtung ein Marker bewegt werden, dessen Bewegung durch den Sensor detektiert wird, um das Bewegungssignal zu erzeugen. Diese Ausführungsvariante hat den Vorteil, dass ebenfalls die Bewegungsüberwachungsvorrichtung mit dem externen Sensor überwacht wird. Es ist jedoch auch denkbar, das Bewegungssignal intern in einer Rechnereinheit zu erzeugen, so dass das Bewegungssignal ein rein virtuelles Bewegungssignal ist, das nicht mit einem sich physikalisch bewegenden Gegenstand korreliert.

Das Phantom kann insbesondere ein sich bewegendes Phantom sein. Die Bewegung kann durch eine für das Phantom vorgesehene Bewegungsvorrichtung erfolgen. Durch die Bewegung des Phantoms kann die Bewegung des zu bestrahlenden Zielvolumens simuliert werden. Die Bewegungsdetektionsvorrichtung kann in diesem Fall zur Generierung des Bewegungssignals die Bewegung des Phantoms überwachen. Die Bewegung des Phantoms ist insbesondere dann vorteilhaft, wenn bei der Bestrahlung als Kompensationsverfahren für eine Bewegung ein Gating- oder ein Rescanning-Verfahren eingesetzt wird. Die Bewegung kann auch derart ausgestaltet sein, dass sie nicht dem Bewegungssignal entspricht. Der Vorteil hierin liegt, dass dadurch das Muster der Dosisverteilung beeinflusst werden kann. Eine ursprünglich homogen geplante Dosisverteilung kann durch das unterschiedliche Bewegungsmuster eine nicht-homogene Dosisverteilung im Phantom erzeugen. Diese kann gegebenenfalls einfacher auszuwerten sein.

Die Bewegungsvorrichtung kann derart gesteuert werden, dass kritische Bewegungstrajektorien simuliert werden, z.B. Bewegungstrajektorien, die eine maximale Beanspruchung des Bewegungskompensationssystems hervorrufen. Die Bewegungstrajektorien können zum Beispiel die abrupte Änderung eines Bewegungszustandes (zum Beispiel durch Husten), einen stetigen Drift der Base-Line des Bewegungssystems, oder eine Bewegung in einem Umfang, der die ursprüngliche Bestrahlungsplanung übersteigt, sodass z.B. der Umfang von Look-Up-Tabellen, in denen Kompensationsparameter hinterlegt sind, oder Sicherheitssäume überschritten werden, simulieren. Bei derartigen Bewegungstrajektorien kann zusätzlich das Interlocksystem der Anlage geprüft werden, das eine Bestrahlung bei derart kritischen Fällen unterbricht.

Das Phantom muss jedoch nicht zwangsläufig ein sich bewegendes Phantom sein, um die Qualität der Bewegungskompensation zu überprüfen. Das Phantom kann insbesondere ein anderes Bewegungsmuster aufweisen, als es durch das nachgebildete Bewegungssignal wiedergegeben ist. So kann das Phantom selbst ein anderes Bewegungsmuster aufweisen als ein Bewegungsphantom, dessen Bewegung zur Nachbildung des Bewegungssignals detektiert wird. Ein Phantom mit einem derartigen Bewegungsmuster wird in Verbindung mit einem Tracking-Verfahren eingesetzt, bei dem der Partikelstrahl durch das Bewegungssignal gesteuert in seiner örtlichen Position abgelenkt wird. In vorteilhafter Weise kann das Phantom sogar ein statisches Phantom sein, obwohl bei dem Verfahren eine Bewegungskompensation überprüft wird.

Diese Ausführungsform beruht auf der Tatsache, dass sogenannte Interferenzeffekte entstehen, wenn sich ein Zielvolumen während der Bestrahlung bewegt und wenn der Strahl nicht der Bewegung nachgeführt würde. Dadurch käme es an Stellen im Zielvolumen zu einer ungewünschten Über- bzw. Unterdosierung in Bezug auf die geplante Dosisverteilung. Gerade das Tracking, das zur Vermeidung von Interferenzeffekten im bewegenden Zielvolumen eingesetzt wird, führt dann allerdings zu ähnlichen "inversen" Interferenzeffekten, wenn ein Phantom bestrahlt wird, das einem anderen Bewegungsmuster unterliegt als im Bewegungssignal wiedergegeben.

Die Kombination eines Phantoms, das ein anderes Bewegungsmuster hat als das durch das Bewegungssignal gegebene, mit dem Tracking-Verfahren führt zu einer Dosisverteilung in dem Phantom, welche ein charakteristisches Verteilungsmuster aufweist. Eine ursprünglich geplante homogene Dosisverteilung beispielsweise erzeugt in dem Phantom eine inhomogene Dosisverteilung. Diese inhomogene Dosisverteilung kann mit einer zu erwartenden inhomogene Dosisverteilung, die aus Daten berechnet wird, die in Zusammenhang mit der Bewegungskompensation während der Bestrahlung stehen, verglichen werden.

Dieser Ausführungsvariante liegt die Erkenntnis zu Grunde, dass gerade die inhomogene Dosisverteilung besonders sensitiv gegenüber Fehler im Bewegungskompensationssystem bei der Bestrahlung ist und Abweichungen leichter zu detektieren sind als bei einer homogenen Dosisverteilung. Der Vergleich des ermittelten mit dem zu erwartenden inhomogenen Musters der Dosisverteilung ist besonders effizient, um etwaige Fehler im Bestrahlungssystem und im Bewegungskompensationssystem zu erkennen.

Die zur Berechnung der zu erwartenden Dosisverteilung verwendeten Parameter können Steuerparameter, die in dem Bestrahlungsplanungsdatensatz hinterlegt sind, und das Bewegungssignal umfassen. Prinzipiell sind diese Parameter ausreichend, die zu erwartende Dosisverteilung zu berechnen. Anhand dieser Parameter kann z.B. eine Sequenz ermittelt werden, die den Bestrahlungsverlauf vorhersagt und die angibt, wann und wo welche Teildosis zu deponieren ist, also wann sich der Partikelstrahl wo befindet, so dass mit der Sequenz aus der spezifischer Bewegungstrajektorie und den spezifischen Bestrahlungszeiten die gesamte zu erwartende Dosisverteilung berechnet werden kann.

Mit Vorteil fließen jedoch Daten in die Berechnung ein, die die Eigenschaft des applizierenden Behandlungsstrahls im Verlauf der Zeit während der Bestrahlung kennzeichnen. Diese Daten können z.B. im Verlauf der Bestrahlung protokolliert werden. Dies kann beispielsweise die aktuelle aus Position des Strahls (x, y, z) mit den aktuellen Kompensationsparametern (dx, dy, dz) sein, die aktuelle Teilchenzahl (N) des Behandlungsstrahls mit eventuellen Kompensationsparameter (dN), insbesondere die pro Zielpunkt applizierte aktuelle Teilchenzahl, und/oder eine Intensität des Behandlungsstrahls, so dass aus diesen den aktuellen Bestrahlungsverlauf kennzeichnenden Daten die zu erwartende Dosisverteilung ermittelt werden kann. Diese Ausführung hat den Vorteil, dass sie nicht wie bei der Vorhersage des Bestrahlungsverlaufs auf Annahmen beruht, die gegebenenfalls nicht der Realität entsprechen. Mit dieser Ausführungsform können Unsicherheiten und Grenzen des Bewegungskompensationssystems der Anlage wie sie tatsächlich vorliegen mit erfasst werden.

Die Berechnung der zu erwartenden Dosisverteilung berücksichtigt ebenfalls den Aufbau des Phantoms, da der Aufbau des Phantoms insbesondere bei Partikelstrahlen mit der Eindringtiefe eines Strahls verbunden ist. So ist beispielsweise bei einem inhomogen aufgebauten Phantom eine andere Dosisverteilung zu erwarten als bei einem homogen aufgebauten Phantom.

In einer vorteilhaften Ausgestaltung werden die Parameter dazu verwendet werden, die Dosisverteilung mit einem dem Bestrahlungsplanungsdatensatz zu Grunde liegenden Abbildungsdatensatz in Beziehung zu setzen. Die Dosisverteilung kann in dem Abbildungsdatensatz, z.B. in einem vierdimensionalen Computertomogramm, rekonstruiert werden. Insbesondere kann so zum Beispiel überprüft werden, ob die zu erwartende Dosisverteilung der Anatomie eines Patienten optimal angepasst ist und wie sich eine Bestrahlung mit Bewegungskompensation im Kontext der Patientenanatomie auswirken würde. Beispielsweise kann geprüft werden, ob die distale Kante des Zielvolumens ausreichend bestrahlt würde oder ob etwaige Risikoorgane ausreichend geschont würden.

Die erfindungsgemäße Vorrichtung zur Überprüfung einer Bestrahlungsanlage, insbesondere einer Partikeltherapieanlage, in der mit einem Behandlungsstrahl eine Dosisverteilung in einem Zielobjekt deponierbar ist, umfasst:
- eine Einrichtung zum Bereitstellen eines Bestrahlungsplanungsdatensatzes, wie z.B. eine Bestrahlungsplanungsvorrichtung oder eine Schnittstelle zum Laden eines Bestrahlungsplans, der für die Bestrahlung eines sich bewegenden Zielvolumens optimiert ist,
- eine Einrichtung zum Bereitstellen eines Bewegungssignals, das eine Bewegung des Zielvolumens nachbildet, wie z.B. eine Bewegungsdetektionsvorrichtung oder eine Rechnereinheit, mit der ein Bewegungssignal erzeugt werden kann,
- ein Phantom, das zur Detektion einer in dem Phantom deponierten Dosisverteilung während oder nach der Bestrahlung ausgebildet ist,
- eine Rechnervorrichtung, die ausgebildet ist zum Berechnen einer zu erwartenden Dosisverteilung in dem Phantom, die mittels der Bestrahlung mit Parametern des Bestrahlungsplanungsdatensatzes erzielt worden ist, also aufgrund von Parametern, die im Zusammenhang mit der Steuerung der Bestrahlungsanlage während der Bestrahlung stehen, und zum Vergleichen einer im Phantom deponierten ermittelten Dosisverteilung mit der berechneten zu erwartenden Dosisverteilung.

Das Ergebnis des Vergleichs kann einem Anwender präsentiert werden, der dann entscheiden kann, welche Maßnahmen zu treffen sind. Denkbar ist eine Wartung der Anlage oder eine Anpassung bzw. Änderung des Bestrahlungsplanungsdatensatzes. Beispielsweise kann ein Signal ausgegeben werden, wenn der Vergleich eine Abweichung feststellt, die oberhalb eines Schwellenwertes liegt.

Die erfindungsgemäße Bestrahlungsanlage, insbesondere Partikeltherapieanlage, weist eine derartige Vorrichtung zur Überprüfung einer Bestrahlungsanlage auf. Die Steuerungsvorrichtung der Bestrahlungsanlage zur Steuerung einer Bestrahlung umfasst ein Bewegungskompensationssystem, sodass mit der Bestrahlungsanlage auch bewegte Zielvolumina bestrahlt werden können.

Ausführungsformen der Erfindung mit Weiterbildungen gemäß den Merkmalen der abhängigen Ansprüche werden anhand der folgenden Zeichnung näher erläutert ohne jedoch darauf beschränkt zu sein. Es zeigen:
Fig. 1 einen schematischen Aufbau einer Partikeltherapieanlage, in der die Qualitätsüberprüfung eines Bestrahlungsverfahrens mit Bewegungskompensation gemäß einer ersten Ausführungsvariante durchgeführt wird,
Fig. 2, Fig. 3 und Fig. 4 einen schematischen Aufbau einer Partikeltherapieanlage, in der die Qualitätsüberprüfung gemäß einer zweiten, dritten bzw. vierten Ausführungsvariante durchgeführt wird, und
Fig. 5 ein Ablaufdiagramm verschiedener Verfahrensschritte, die bei einer Ausführungsform des erfindungsgemäßen Verfahrens ausgeführt werden können.

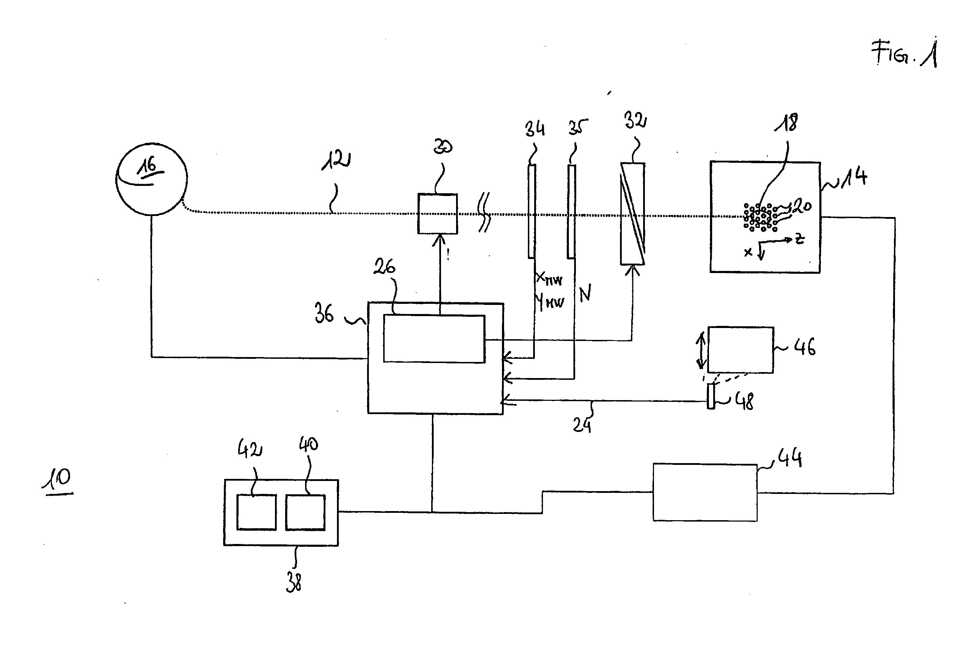

Fig. 1 zeigt in stark schematisierter Darstellung einen Aufbau einer Partikeltherapieanlage 10. Die Partikeltherapieanlage 10 wird zur Bestrahlung eines auf einer Positioniervorrichtung angeordneten Körpers mit einem Strahl aus Partikeln eingesetzt, der im Folgenden als Partikelstrahl 12 bezeichnet ist. Insbesondere kann als Zielvolumen ein tumorerkranktes Gewebe eines Patienten mit dem Partikelstrahl 12 bestrahlt werden. Zur Qualitätsüberprüfung für ein Bestrahlungsverfahren, bei dem eine Bewegungskompensation eingesetzt wird, um die die Applikation der Dosisverteilung an eine Bewegung des Zielvolumens anzupassen, wird jedoch ein Phantom 14 bestrahlt.

In dem zu bestrahlenden Phantom 14 wird die Dosisverteilung in einem Zielbereich 18 deponiert, der im Allgemeinen dem Zielvolumen, das in einem Bestrahlungsplanungsdatensatz definiert ist, entspricht. Im Phantom sind mehrere Detektoren 20 zur räumlich aufgelösten Detektion der Dosisverteilung verteilt. Nach oder während der Bestrahlung des Phantoms 14 kann so die tatsächlich deponierte Dosisverteilung räumlich aufgelöst ermittelt werden.

Die Partikeltherapieanlage 10 weist typischerweise eine Beschleunigereinheit 16 auf, z.B. ein Synchrotron, ein Zyklotron oder einen sonstigen Beschleuniger, der einen Partikelstrahl 12 mit der zur Bestrahlung notwendigen Energie bereitstellt. Als Partikel werden vornehmlich Teilchen wie beispielsweise Protonen, Pionen, Heliumionen, Kohlenstoffionen oder Ionen anderer Elemente eingesetzt. Typischerweise hat ein Partikelstrahl 12 einen Strahldurchmesser von 3-10 mm. Der Partikelstrahl wird im Allgemeinen über das Zielvolumen gescannt.

Als Scanverfahren wird bevorzugt ein Rasterscan-Verfahren verwendet, bei dem der Partikelstrahl 12 sukzessive an verschiedene Zielpunkte im Phantom gelenkt wird und dort jeweils die im Bestrahlungsplanungsdatensatz hinterlegte Teildosis appliziert. Der Partikelstrahl 12 wird von Zielpunkt zu Zielpunkt gelenkt ohne zwangsläufige Abschaltung zwischen den einzelnen Zielpunkten. Es können auch Spotscan-Verfahren mit Abschaltung des Partikelstrahls 12 zwischen den einzelnen Zielpunkten oder andere Scan-Verfahren wie beispielsweise kontinuierliche Scanverfahren eingesetzt werden, um den Zielbereich mit dem Partikelstrahl 12 zu bestrahlen.

Der Partikelstrahl 12 wird in seiner lateralen Auslenkung typischerweise mithilfe eines Systems von Scan-Magneten 30 beeinflusst, d.h. in seiner Position senkrecht zu Strahlverlaufsrichtung, die auch als x- und y-Richtung bezeichnet wird, abgelenkt. Weiterhin kann eine Energiemodulationsvorrichtung 32 vorgesehen sein, mit der die Energie des Partikelstrahls 12 schnell geändert werden kann, so dass die Eindringtiefe des Partikelstrahls 12 variiert und damit der Ort des Bragg-Peaks in z-Richtung verändert werden kann.

Auf diese Weise ist es möglich, ein Tracking-Verfahren zu implementieren. Dies bedeutet, dass bei der Bestrahlung des Zielvolumens der aktuelle Ort des Partikelstrahls (x, y, z) um Korrekturwerte (dx, dy, dz) und gegebenen falls die pro Zielpunkt zu applizierende Teilchenzahl (N) um Korreturwerte (dN) verändert werden, welche aufgrund eines Bewegungssignals gesteuert werden und welche die Abweichung von der Idealposition aufgrund eines sich bewegenden Zielvolumens ausgleichen.

Weiterhin weist die Anlage Ortsdetektoren 34 und Intensitätsdetektoren 35 zur Überwachung der Strahlparameter wie die tatsächliche laterale Ablenkung (x_{MW}, y_{MW}) und die tatsächlich applizierten Teilchenzahlen (N) auf.

Die Bestrahlungsanlage 10 und insbesondere der Bestrahlungsvorgang werden durch eine Steuerungsvorrichtung 36 gesteuert. Die Steuerungsvorrichtung 36, mit deren Hilfe ein Bestrahlungsplanungsdatensatz 40 geladen und zur spezifischen Steuerung der Bestrahlungsanlage 10 umgesetzt werden kann, umfasst eine Bewegungskompensationsvorrichtung, die in diesem Fall als Strahlnachführungseinheit 26 ("beam tracking unit") ausgebildet ist. Die Strahlnachführungseinheit 26 sendet Steuerparameter an die Scan-Magnete 30, um den lateralen Ort des Partikelstrahls 12 (x, y) an eine Verschiebung des Zielvolumens anzupassen (dx, dy), und an die Energiemodulationsvorrichtung 32, um den longitudinalen Ort (z) des Bragg-Peaks des Partikelstrahls 12 anzupassen (dz). Gegebenenfalls werden auch die in einem Zielpunkt zu applizierenden Teilchenzahlen (N) angepasst (dN). Die Kompensationsparameter werden mithilfe eines vierdimensionalen Bestrahlungsplanungsdatensatzes 40 und mithilfe eines Bewegungssignals 24 während der Bestrahlung ermittelt.

Die Steuerungsvorrichtung 36 protokolliert die mit den Detektoren 34, 35 aufgezeichneten Daten, sodass hieraus rekonstruiert werden kann, wann der Partikelstrahl 12 an welchem Ort und wie intensiv appliziert worden ist.

Üblicherweise erfolgt die Steuerung basierend auf einem Bestrahlungsplan bzw. auf einem Bestrahlungsplanungsdatensatz 40, der mithilfe einer Bestrahlungsplanungseinrichtung 38 ermittelt und bereitgestellt wird. Der hier gezeigte Bestrahlungsplanungsdatensatz 40 wurde für einen (nicht gezeigten) Patienten erstellt, dessen zu bestrahlendes Zielvolumen sich bewegt. Der Bestrahlungsplanungsdatensatz 40 berücksichtigt diese Bewegung und kann deshalb als vierdimensionaler Bestrahlungsplanungsdatensatz 40 bezeichnet werden. Er wird üblicherweise anhand eines einem vierdimensionalen Computertomogramms 42 erstellt.

Die Partikeltherapieanlage 10 weist zudem eine Rechnereinheit 44 auf, mit der die durch die Bestrahlung des Phantoms 18 detektierte Dosisverteilung mit einer zu erwartenden Dosisverteilung, die aus Daten des Bewegungssignals 24, des vierdimensionalen Bestrahlungsplanungsdatensatzes 40 und der protokollierten Strahlparameter berechnet wird, verglichen werden kann. Die Rechnereinheit 44 ist hier der Übersichtlichkeit halber als separate Einheit gezeigt. Die durch die Rechnereinheit 44 ausgeführte Funktionalität kann jedoch auch in bereits bestehende Komponenten, insbesondere in Steuerungskomponenten einer Partikeltherapieanlage 10 implementiert werden.

Bei zu großer Abweichung der durch das Phantom 14 ermittelten Dosisverteilung von der berechneten Dosisverteilung wird ein Signal ausgegeben, das einem Anwender anzeigt, dass die Bestrahlungsanlageanlage 10 und/oder die Bestrahlungsplanung 40 verändert werden muss, bevor der Bestrahlungsplanungsdatensatz 40 an einem Patienten umgesetzt werden kann. Die Korrektur kann dann im besten Fall automatisch durchgeführt werden, im Regelfall wird jedoch ein Anwender eine neue Dosisverteilung erstellen und/oder manuell bewerten.

In Fig. 1 ist das Phantom 14 statisch ausgebildet. Die Bewegung wird simuliert, indem ein eigens vorgesehenes Bewegungsphantom 46 durch eine nicht gezeigte Bewegungsvorrichtung bewegt wird, sodass ein Bewegungsdetektionsvorrichtung 48 eine Bewegung detektieren und ein Bewegungssignal 24 erzeugen kann.

Mithilfe dieses Bewegungssignals 24 führt die Steuerungsvorrichtung 36 und die Strahlnachführungseinheit 26 eine Nachführung des Partikelstrahls 12 durch, als ob sich das zu bestrahlende Phantom 14 gemäß dem Bewegungssignal 24 bewegen würde.

Die in dem statischen Phantom 14 deponierte Dosisverteilung weist aufgrund der Bewegungsnachführung des Partikelstrahls 12 ein charakteristisches Dosisverteilungsmuster auf. Dieses Dosisverteilungsmuster ist besonders sensitiv gegenüber Abweichungen von einer geplanten Bestrahlung. Durch Vergleich des gemessenen Dosisverteilungsmusters mit dem berechneten Dosisverteilungsmuster können Ungenauigkeiten bei der Bestrahlung besonders sensitiv detektiert werden.

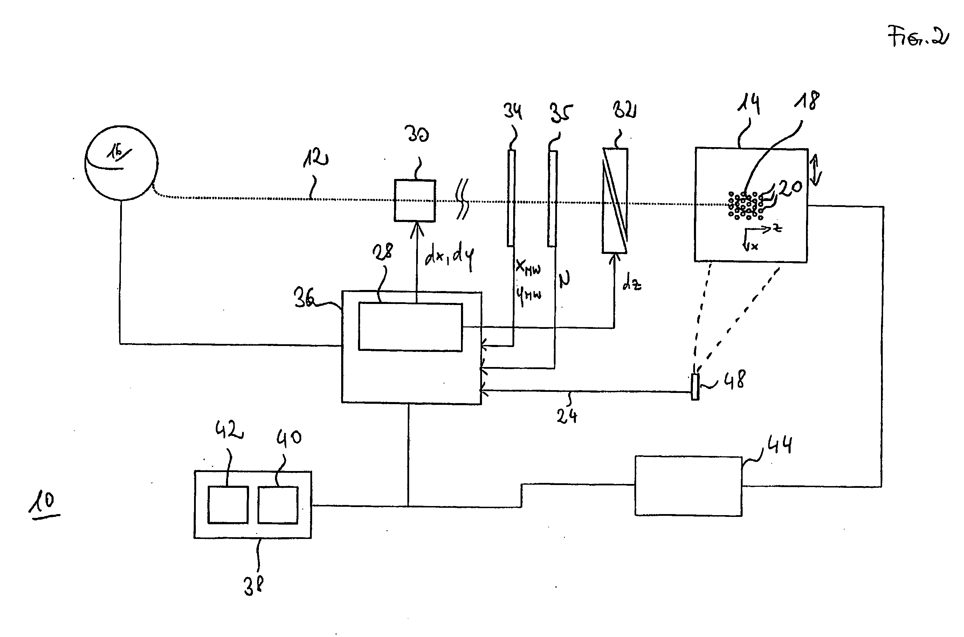

Fig. 2 unterscheidet sich von der in Fig. 1 gezeigten Partikeltherapieanlage 10 dadurch, dass nun das Phantom 14 selbst durch eine nicht gezeigte Bewegungsvorrichtung bewegt wird. Die Bewegung des Phantoms 14 wird mit der Bewegungsdetektionsvorrichtung 48 detektiert, und hierauf basierend steuert die Steuerungsvorrichtung 36 den Bestrahlungsvorgang. Die Bewegungskompensationsvorrichtung 28 ist in Fig. 2 jedoch derart ausgebildet, das die Bewegungskompensation mithilfe eines Gating-Verfahrens oder mithilfe eines Rescanning-Verfahrens durchgeführt wird. Das Gating-Verfahren beruht auf einer Anpassung der Bestrahlung (An- und Abschaltung) anhand des detektierten Bewegungssignals 24. Je nach Ausgestaltung kann auch das Rescanning-Verfahren auf einer Anpassung der Bestrahlung, z.B. der einzelnen Rescan-Durchgänge anhand des detektierten Bewegungssignals beruhen.

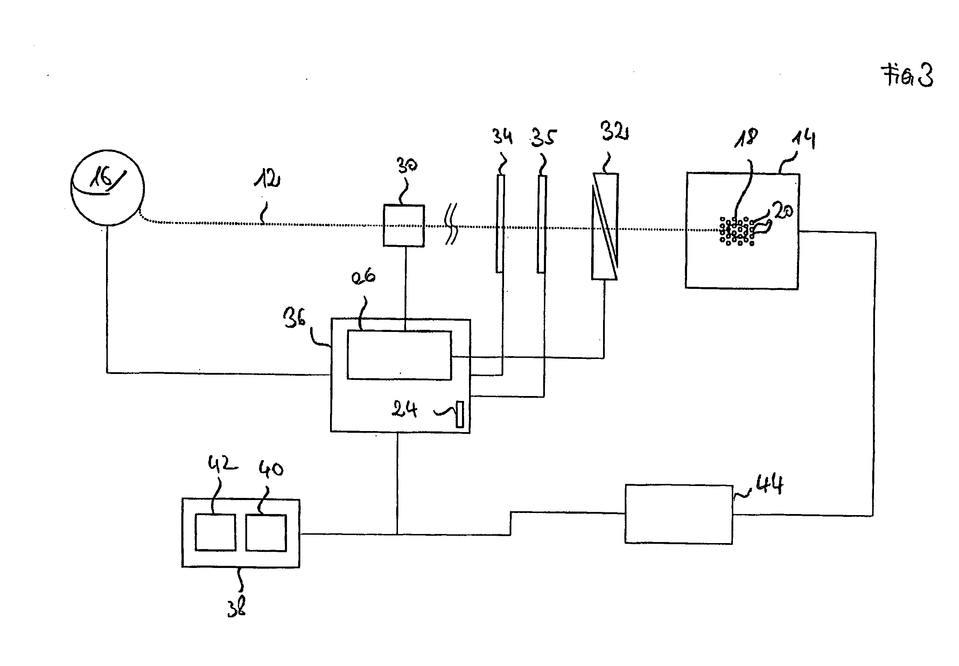

Fig. 3 unterscheidet sich von der in Fig. 1 gezeigten Partikeltherapieanlage 10 dadurch, dass nun auf die Bewegungsdetektionsvorrichtung bei der Qualitätsüberprüfung verzichtet wird. Das Bewegungssignal 24 wird hier intern in der Steuerungsvorrichtung 36 generiert und ist ein rein virtuelles Signal 24, das beispielsweise die Schwerpunktsbewegung eines Zielvolumens nachbildet. Diese Ausführungsvariante hat den Nachteil, dass etwaige Fehlerquellen, die durch die Bewegungskompensationsvorrichtung bedingt sein können, nicht erfasst werden. Sie hat den Vorteil, dass kein Bewegungsphantom oder eigene Messvorrichtung zum Generieren des Bewegungssignals 24 benötigt wird.

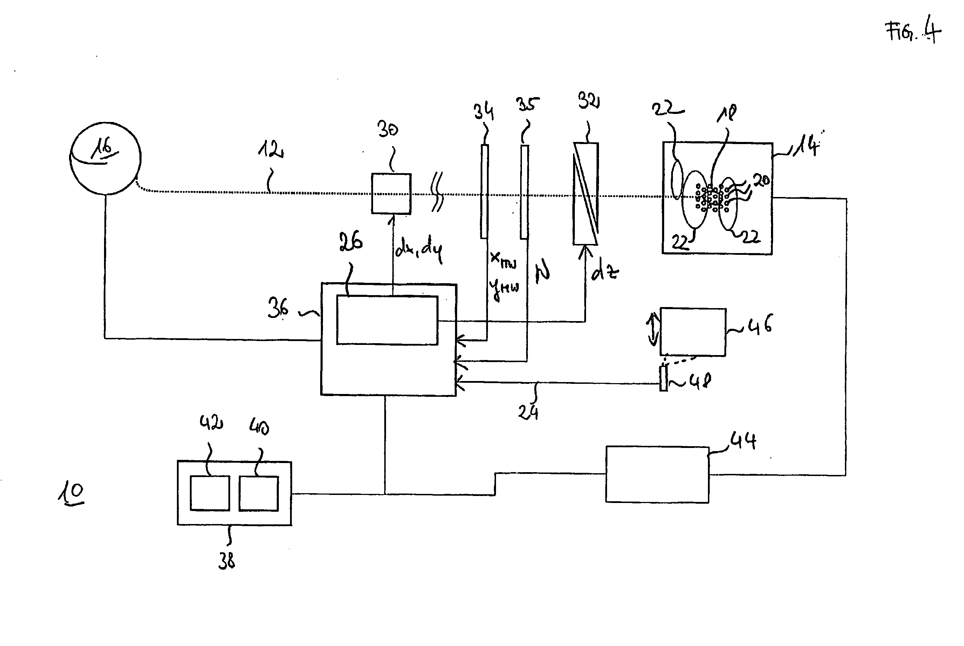

Fig. 4 unterscheidet sich von der Ausführungsform gemäß Fig. 1 dadurch, dass das gezeigte Phantom 14 mehrere unterschiedliche Bereiche 22 aufweist, in die der Partikelstrahl 12 unterschiedlich weit eindringt. Mit einem derartigen Phantom 14 lässt sich zum Beispiel die Anatomie eines Patienten nachbilden, um realistischere Bestrahlungsszenarien überprüfen zu können. Bei der Berechnung der Dosisverteilung werden die unterschiedlichen Eindringtiefen im Phantom 14 berücksichtigt. Dies kann z.B. anhand eines Computertomogramms des Phantoms 14 erfolgen, anhand dessen man die Reichweite des Partikelstrahls 12 im Phantom 14 bestimmen kann.

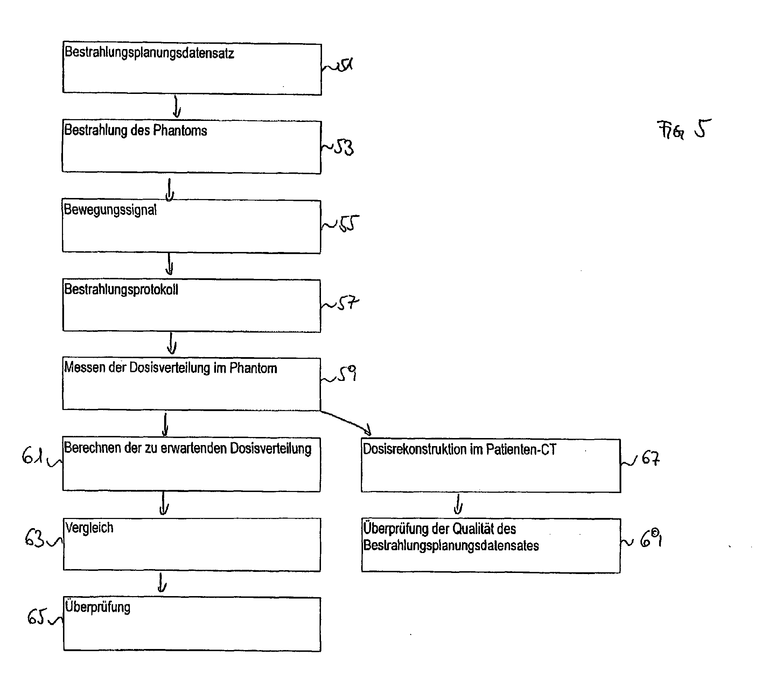

Fig. 5 zeigt einen schematischen Überblick über verschiedene Verfahrensschritte, die bei einer Ausführungsform des Verfahrens durchgeführt werden können.

Zunächst wird ein Bestrahlungsplanungsdatensatz bereitgestellt, der auf einem 4DCT eines Patienten beruht (Schritt 51).

Dieser Bestrahlungsplan wird jedoch zunächst dazu verwendet, ein Phantom zu bestrahlen (Schritt 53), basierend auf einem bereitgestellten simulierten Bewegungssignal (Schritt 55).

Während der Bestrahlung werden die charakteristischen Strahlparameter, die die Bestrahlung kennzeichnen, wie der zeitliche Verlauf des Ortes und/oder der Ablenkung des Partikelstrahls und/oder anderer Eigenschaften des Partikelstrahls wie die Intensität, die aktuelle Teilchenzahl oder der Fokus aufgezeichnet und ein Bestrahlungsprotokoll erstellt (Schritt 57).

Anschließend wird die Dosisverteilung, die bei der Bestrahlung im Phantom appliziert worden ist, gemessen bzw. ermittelt (Schritt 59).

Anschließend wird die zu erwartende Dosisverteilung in dem Phantom berechnet, die mittels der Bestrahlung anhand von Parametern des Bestrahlungsplanungsdatensatzes durchgeführt wurde, also basierend auf dem Bestrahlungsplanungsdatensatz, auf dem während der Bestrahlung verwendeten Bewegungssignal sowie auf den während der Bestrahlung protokollierten Mess- bzw. Steuerdaten (Schritt 61). Die Berechnung der Dosisverteilung kann eine Abbildung des Phantoms, z.B. ein Computertomographen des Phantoms, mit einbeziehen.

Die berechnete zu erwartende Dosisverteilung wird mit der tatsächlich ermittelten und gemessenen Dosisverteilung des Phantoms verglichen (Schritt 63). Hierdurch kann eine Überprüfung des Bestrahlungssystems bzw, der Partikeltherapieanlage und/oder des Bestrahlungsplanungsdatensatzes (Schritt 65) erfolgen.

In einem weiteren optionalen Schritt kann unter Verwendung des Bestrahlungsplanungsdatensatzes, des während der Bestrahlung verwendeten Bewegungssignals sowie der während der Bestrahlung protokollierten Mess- bzw. Steuerdaten eine Dosisverteilung in einem Patienten-Computertomogramm berechnet werden, und zwar in dem (zeitaufgelösten) Computertomogramm, das dem Bestrahlungsplanungsdatensatz zu Grunde gelegen hat (Schritt 67).

Durch diese Berechnung kann festgestellt werden, wie bei einer tatsächlichen Bestrahlung sich die Dosisverteilung auf einen Patienten auswirken würde. Auf diese Weise kann die Qualität einer geplanten Bestrahlung bezogen auf die tatsächlich vorliegenden anatomischen Verhältnisse überprüft werden (Schritt 69).

### Bezugszeichenliste

- 10: Partikeltherapieanlage
- 12: Partikelstrahl
- 14: Phantom
- 16: Beschleunigereinheit
- 18: Zielbereich
- 20: Detektoren
- 22: unterschiedliche Bereiche
- 24: Bewegungssignal
- 26: Strahlnachführungseinheit
- 28: Bewegungskompensationsvorrichtung
- 30: System von Scan-Magneten
- 32: Energiemodulationsvorrichtung
- 34: Ortsdetektor
- 35: Intensitätsdetektor
- 36: Steuerungsvorrichtung
- 38: Bestrahlungsplanungseinrichtung
- 40: Bestrahlungsplanungsdatensatz
- 42: Computertomograph
- 44: Rechnereinheit
- 46: Bewegungsphantom
- 48: Bewegungsdetektionsvorrichtung

- 51: Schritt 51
- 53: Schritt 53
- 55: Schritt 55
- 57: Schritt 57
- 59: Schritt 59
- 61: Schritt 61
- 63: Schritt 63
- 65: Schritt 65
- 67: Schritt 67
- 69: Schritt 69

## Patentansprüche

1. Verfahren zur Überprüfung einer Bestrahlungsplanung bei einer Partikeltherapieanlage, welche als Partikel Protonen, Pionen, Heliumionen, Kohlenstoffionen oder Ionen anderer Elemente einsetzt, in der mit einem Partikelbehandlungsstrahl (12) eine Dosisverteilung in einem Zielobjekt (14) mit einem Gating-Verfahren, bei dem der Partikelbehandlungsstrahl gesteuert durch ein Bewegungssignal (24) aktiviert bzw. deaktiviert wird, mit einem Rescanning-Verfahren, bei dem die Dosisverteilung in einem Zielvolumen des Zielobjekts durch mehrfach sukzessives Applizieren von Teildosen an gleichem Ort aufgebaut wird oder mit einem Tracking-Verfahren, bei dem der Partikelbehandlungsstrahl einer Bewegung des Zielvolumens nachgeführt wird, deponierbar ist, umfassend folgende Schritte:
- Bereitstellen eines Bestrahlungsplanungsdatensatzes (40), der für die Bestrahlung des sich bewegenden Zielvolumens erstellt ist, wobei in dem Bestrahlungsplanungsdatensatz (40) Steuerparameter zur Steuerung der Partikeltherapieanlage hinterlegt sind,
- Bereitstellen des Bewegungssignals (24), das eine Bewegung des Zielvolumens nachbildet,
- Bestrahlen eines Phantoms (14) unter Verwendung der im Bestrahlungsplanungsdatensatz (40) hinterlegten Steuerparameter und des Bewegungssignals (24), wobei das Phantom (14) zur Detektion einer in dem Phantom deponierten Dosisverteilung während oder nach der Bestrahlung ausgebildet ist,
- Ermitteln einer im Phantom (14) deponierten Dosisverteilung,
- Berechnen einer zu erwartenden Dosisverteilung aufgrund von Parametern, die im Zusammenhang mit der Steuerung der Partikeltherapieanlage (10) während der Bestrahlung stehen,
- Vergleichen der im Phantom (14) deponierten ermittelten Dosisverteilung mit der berechneten zu erwartenden Dosisverteilung.

2. Verfahren nach Anspruch 1, wobei das Phantom (14) ein sich bewegendes Phantom ist.

3. Verfahren nach einem der Ansprüche 1 oder 2, wobei das Phantom (14) ein anderes Bewegungsmuster aufweist als das Bewegungssignal (24).

4. Verfahren nach Anspruch 3, bei dem der Vergleich anhand eines im Phantom (14) deponierten inhomogenen Musters in der Dosisverteilung mit einem zu erwartenden inhomogenen Muster durchgeführt wird.

5. Verfahren nach einem der vorherigen Ansprüche, bei dem die zur Berechnung der zu erwartenden Dosisverteilung verwendeten Parameter Steuerparameter, die in dem Bestrahlungsplanungsdatensatz (40) hinterlegt sind, und das Bewegungssignal (24) umfassen.

6. Verfahren nach einem der vorherigen Ansprüche, bei dem die zur Berechnung der zu erwartenden Dosisverteilung verwendeten Parameter Daten sind, die eine aktuelle Eigenschaft des Partikelbehandlungsstrahls (12) während der Bestrahlung, insbesondere eine Lage (x, y, z, dx, dy, dz) des Partikelbehandlungsstrahls (12) und/oder eine applizierte Teilchenzahl des Partikelbehandlungsstrahls (12) während der Bestrahlung kennzeichnen.

7. Verfahren nach Anspruch 6, bei dem die Parameter dazu verwendet werden, die Dosisverteilung mit einem dem Bestrahlungsplanungsdatensatz (40) zu Grunde liegenden Abbildungsdatensatz (42) in Beziehung zu setzen.

8. Verfahren nach einem der vorherigen Ansprüche, wobei das Bewegungssignal (24) ein intern in einer Rechnereinheit generiertes virtuelles Bewegungssignal (24) ist.

9. Verfahren nach einem der Ansprüche 1 bis 7, bei dem das Bewegungssignal (24) ein durch eine Bewegungsdetektionsvorrichtung (48) erfasstes Bewegungssignal (24) ist.

10. Verfahren nach einem der vorherigen Ansprüche, bei dem das Phantom (14) ein 3D-Phantom ist.

11. Verfahren nach Anspruch 10, bei dem das Phantom (14) mehrere unterscheidbare Bereiche (22) aufweist, die aus Materialien mit unterschiedlicher Eindringtiefe für einen Partikelstrahls bestehen, wobei das Phantom (14) insbesondere anthropomorph aufgebaut ist.

12. Verfahren nach einem der Ansprüche 10 oder 11, wobei bei der Berechnung der zu erwartenden Dosisverteilung der Aufbau des Phantoms (14) berücksichtigt wird.

13. Vorrichtung zur Überprüfung einer Bestrahlungsplanung (10) bei einer Partikeltherapieanlage, welche als Partikel Protonen, Pionen, Heliumionen, Kohlenstoffionen oder Ionen anderer Elemente einsetzt, in der mit einem Partikelbehandlungsstrahl (12) eine Dosisverteilung in einem Zielobjekt mit einem Gating-Verfahren, bei dem der Partikelbehandlungsstrahl gesteuert durch ein Bewegungssignal (24) aktiviert bzw. deaktiviert wird, mit einem Rescanning-Verfahren, bei dem die Dosisverteilung in einem Zielvolumen des Zielobjekts durch mehrfach sukzessives Applizieren von Teildosen an gleichem Ort aufgebaut wird oder mit einem Tracking-Verfahren, bei dem der Partikelbehandlungsstrahl einer Bewegung des Zielvolumens nachgeführt wird, deponierbar ist, umfassend:
- eine Einrichtung (38) zum Bereitstellen eines Bestrahlungsplanungsdatensatzes (40), der für die Bestrahlung des sich bewegenden Zielvolumens optimiert ist, wobei in dem Bestrahlungsplanungsdatensatz (40) Steuerparameter zur Steuerung der Partikeltherapieanlage (10) hinterlegt sind,
- eine Einrichtung (48) zum Bereitstellen des Bewegungssignals (24), das eine Bewegung des Zielvolumens nachbildet,
- ein Phantom (14), das zur Detektion einer in dem Phantom (14) deponierten Dosisverteilung während oder nach der Bestrahlung ausgebildet ist,
eine Rechnervorrichtung (44) zum Berechnen einer zu erwartenden Dosisverteilung aufgrund von Parametern, die im Zusammenhang mit der Steuerung der Partikeltherapieanlage (10) während der Bestrahlung stehen, und zum Vergleichen einer im Phantom (14) deponierten ermittelten Dosisverteilung mit der berechneten zu erwartenden Dosisverteilung.

14. Partikeltherapieanlage (10), mit
einer Bestrahlungsplanungseinrichtung (38),
einer Vorrichtung zur Überprüfung einer Bestrahlungsplanung der Bestrahlungsplanungseinrichtung (38) nach Anspruch 13 und
eine Signalausgabeeinrichtung zur Anzeige des mit der Rechnervorrichtung (44) berechneten Vergleichs der in dem Phantom (14) deponierten ermittelten Dosisverteilung mit der berechneten zu erwartenden Dosisverteilung.

## Claims

1. Method for checking irradiation planning in a particle therapy system using protons, pions, helium ions, carbon ions or ions of other elements as particles, in which a dose distribution is depositable in a target object (14) with a particle therapy beam (12) using a gating method, in which the particle therapy beam is activated and deactivated in a controlled manner by way of a motion signal (24), using a rescanning method, in which the dose distribution in a target volume of the target object is built up by multiple successive applications of partial doses at the same site, or using a tracking method, in which the particle therapy beam tracks a movement of the target volume, comprising the following steps:
- providing an irradiation planning data set (40), which is created for irradiating the moving target volume, wherein control parameters for controlling the particle therapy system are stored in the irradiation planning data set (40),
- providing the motion signal (24) which simulates a movement of the target volume,
- irradiating a phantom (14) using the control parameters stored in the irradiation planning data set (40) and the motion signal (24), wherein the phantom (14) is configured for detecting a dose distribution deposited in the phantom during or after the irradiation,
- ascertaining a dose distribution deposited in the phantom (14),
- calculating an expected dose distribution on the basis of parameters which relate to the control of the particle therapy system (10) during the irradiation,
- comparing the ascertained dose distribution deposited in the phantom (14) to the calculated expected dose distribution.

2. Method according to Claim 1, wherein the phantom (14) is a moving phantom.

3. Method according to either of Claims 1 and 2, wherein the phantom (14) has a different movement pattern than the motion signal (24).

4. Method according to Claim 3, in which the comparison is carried out between a non-homogeneous pattern deposited in the phantom (14) in the dose distribution and an expected non-homogeneous pattern.

5. Method according to one of the preceding claims, in which the parameters used to calculate the expected dose distribution comprise control parameters which are stored in the irradiation planning data set (40) and the motion signal (24).

6. Method according to one of the preceding claims, in which the parameters used to calculate the expected dose distribution are data which characterize a current property of the particle therapy beam (12) during the irradiation, in particular a position (x, y, z, dx, dy, dz) of the particle therapy beam (12) and/or an applied number of particles of the particle therapy beam (12) during the irradiation.

7. Method according to Claim 6, in which the parameters are used to correlate the dose distribution to an imaging data set (42) on which the irradiation planning data set (40) is based.

8. Method according to one of the preceding claims, wherein the motion signal (24) is a virtual motion signal (24) generated internally in a computer unit.

9. Method according to one of Claims 1 to 7, in which the motion signal (24) is a motion signal (24) captured by a motion detection apparatus (48).

10. Method according to one of the preceding claims, in which the phantom (14) is a 3D phantom.

11. Method according to Claim 10, in which the phantom (14) has a plurality of distinguishable regions (22) consisting of materials having different penetration depths for a particle beam, wherein the construction of the phantom (14) is in particular anthropomorphic.

12. Method according to either of Claims 10 and 11, wherein the construction of the phantom (14) is taken into account when calculating the expected dose distribution.

13. Apparatus for checking irradiation planning (10) in a particle therapy system, using protons, pions, helium ions, carbon ions or ions of other elements as particles, in which a dose distribution is depositable in a target object with a particle therapy beam (12) using a gating method, in which the particle therapy beam is activated and deactivated in a controlled manner by way of a motion signal (24), using a rescanning method, in which the dose distribution in a target volume of the target object is built up by multiple successive applications of partial doses at the same site, or using a tracking method, in which the particle therapy beam tracks a movement of the target volume, comprising:
- a device (38) for providing an irradiation planning data set (40) which is optimized for irradiating the moving target volume, wherein control parameters for controlling the particle therapy system (10) are stored in the irradiation planning data set (40),
- a device (48) for providing the motion signal (24) which simulates a movement of the target volume,
- a phantom (14) which is configured for the detection of a dose distribution deposited in the phantom (14) during or after the irradiation,
- a computer apparatus (44) for calculating an expected dose distribution on the basis of parameters which relate to the control of the particle therapy system (10) during the irradiation, and for comparing an ascertained dose distribution deposited in the phantom (14) to the calculated expected dose distribution.

14. Particle therapy system (10), comprising
an irradiation planning device (38),
an apparatus for checking the irradiation planning of the irradiation planning device (38) according to Claim 13 and
a signal output device for displaying the comparison which is calculated using the computer apparatus (44) of the ascertained dose distribution deposited in the phantom (14) to the calculated expected dose distribution.

## Revendications

1. Procédé de vérification d'un programme de traitement par rayonnement pour une installation de thérapie par particules, qui utilise en tant que particules des protons, des pions, des ions d'hélium, des ions de carbone ou des ions d'autres éléments et dans laquelle un faisceau de traitement par particules (12) permet de déposer une distribution de dose dans un objet cible (14) avec un procédé d'asservissement, selon lequel le faisceau de traitement par particules est activé ou désactivé sous la commande d'un signal de mouvement (24), avec un procédé de balayages multiples, selon lequel la distribution de dose, dans un volume cible de l'objet cible, est mise en place par plusieurs applications successives de doses partielles au même endroit, ou avec un procédé de guidage, selon lequel le faisceau de traitement par particules est guidé pour suivre un mouvement du volume cible, comprenant les étapes suivantes :
- préparation d'un ensemble de données de programme de traitement par rayonnement (40) qui est établi pour l'irradiation du volume cible en mouvement, sachant que des paramètres de commande, destinés à commander l'installation de thérapie par particules, sont déposés dans l'ensemble de données de programme de traitement par rayonnement (40),
- préparation du signal de mouvement (24) qui reproduit un mouvement du volume cible,
- irradiation d'un fantôme (14), en utilisant les paramètres de commande, déposés dans l'ensemble de données de programme de traitement par rayonnement (40), et du signal de mouvement (24), le fantôme (14) étant conçu pour la détection d'une distribution de dose déposée dans le fantôme, pendant ou après l'irradiation,
- détermination d'une distribution de dose déposée dans le fantôme (14),
- calcul d'une distribution de dose attendue, sur la base de paramètres qui sont en relation avec la commande de l'installation de thérapie par particules (10) pendant l'irradiation,
- comparaison de la distribution de dose déterminée, déposée dans le fantôme (14), avec la distribution de dose calculée attendue.

2. Procédé selon la revendication 1, selon lequel le fantôme (14) est un fantôme en mouvement.

3. Procédé selon l'une quelconque des revendications 1 ou 2, selon lequel le fantôme (14) présente un type de mouvement différent de celui du signal de mouvement (24).

4. Procédé selon la revendication 3, selon lequel la comparaison est effectuée à l'aide d'un motif non homogène dans la distribution de dose, déposé dans le fantôme (14), avec un motif non homogène attendu.

5. Procédé selon l'une quelconque des revendications précédentes, selon lequel les paramètres utilisés pour le calcul de la distribution de dose attendue comprennent des paramètres de commande, qui sont déposés dans l'ensemble de données de programme de traitement par rayonnement (40), et le signal de mouvement (24).

6. Procédé selon l'une quelconque des revendications précédentes, selon lequel les paramètres utilisés pour le calcul de la distribution de dose attendue sont des données qui caractérisent une propriété actuelle du faisceau de traitement par particules (12) pendant l'irradiation, en particulier une position (x, y, z, dx, dy, dz) du faisceau de traitement par particules (12) et/ou un nombre de particules appliqué du faisceau de traitement par particules (12) pendant l'irradiation.

7. Procédé selon la revendication 6, selon lequel les paramètres sont utilisés pour mettre la distribution de dose en relation avec un ensemble de données de reproduction (42) servant de base à l'ensemble de données de programme de traitement par rayonnement (40).

8. Procédé selon l'une quelconque des revendications précédentes, selon lequel le signal de mouvement (24) est un signal de mouvement (24) virtuel généré de façon interne dans une unité d'ordinateur.

9. Procédé selon l'une quelconque des revendications 1 à 7, selon lequel le signal de mouvement (24) est un signal de mouvement (24) recueilli par un dispositif de détection de mouvement (48).

10. Procédé selon l'une quelconque des revendications précédentes, selon lequel le fantôme (14) est un fantôme 3D.

11. Procédé selon la revendication 10, selon lequel le fantôme (14) présente plusieurs zones (22) différenciables qui sont constituées de matériaux ayant des profondeurs de pénétration différentes pour un faisceau de particules, le fantôme (14) étant conçu notamment de façon anthropomorphe.

12. Procédé selon l'une quelconque des revendications 10 ou 11, selon lequel le calcul de la distribution de dose attendue tient compte de la structure du fantôme (14).

13. Dispositif de vérification d'un programme de traitement par rayonnement (10) dans une installation de thérapie par particules qui utilise en tant que particules des protons, des pions, des ions d'hélium, des ions de carbone ou des ions d'autres éléments et dans laquelle un faisceau de traitement par particules (12) permet de déposer une distribution de dose dans un objet cible avec un procédé d'asservissement, selon lequel le faisceau de traitement par particules est activé ou désactivé sous la commande d'un signal de mouvement (24), avec un procédé de balayages multiples, selon lequel la distribution de dose, dans un volume cible de l'objet cible, est mise en place par plusieurs applications successives de doses partielles au même endroit, ou avec un procédé de guidage, selon lequel le faisceau de traitement par particules est guidé pour suivre un mouvement du volume cible, comprenant :
- un dispositif (38) de préparation d'un ensemble de données de programme de traitement par rayonnement (40), qui est optimisé pour l'irradiation du volume cible en mouvement, sachant que des paramètres de commande, destinés à commander l'installation de thérapie par particules (10), sont déposés dans l'ensemble de données de programme de traitement par rayonnement (40),
- un dispositif (48) de préparation du signal de mouvement (24) qui reproduit un mouvement du volume cible,
- un fantôme (14) qui est conçu pour la détection d'une distribution de dose déposée dans le fantôme (14), pendant ou après l'irradiation,
- un dispositif d'ordinateur (44) pour calculer une distribution de dose attendue, sur la base de paramètres qui sont en relation avec la commande de l'installation de thérapie par particules (10) pendant l'irradiation, et pour comparer la distribution de dose déterminée, déposée dans le fantôme (14), avec la distribution de dose calculée attendue.

14. Installation de thérapie par particules (10), comprenant
- un dispositif de planification de traitement par rayonnement (38),
- un dispositif de vérification d'un programme de traitement par rayonnement du dispositif de planification de traitement par rayonnement (38) selon la revendication 13, et
un dispositif d'émission de signaux destiné à visualiser la comparaison, calculée avec le dispositif d'ordinateur (44), de la distribution de dose déterminée, déposée dans le fantôme (14), avec la distribution de dose calculée attendue.
